# FASCICULE DE BREVET EUROPEEN

(11) **EP 4 166 653 B1**
(45) Date de publication et mention de la délivrance du brevet: **30.07.2025**
(21) Numéro de dépôt: 22212642.7
(22) Date de dépôt: 15.12.2016
(51) Int. Cl.: A01K 67/0271, C12N 5/073, C12N 5/079, C12N 5/071, C12N 5/09

(54) **MODÈLE D'ÉTUDE ANIMAL DU CANCER**
TIERMODELL FÜR KREBS
ANIMAL MODEL FOR CANCER STUDY

(30) Priorité: 17.12.2015 FR 1562693
(43) Date de publication de la demande: 19.04.2023
(62) Demande divisionnaire de: 16809852.3
(73) Titulaire: Université Claude Bernard Lyon 1, 69100 Villeurbanne (FR); Centre National de la Recherche Scientifique, 75016 Paris (FR)
(72) Inventeur: CASTELLANI-LINCONTANG, Valérie, 69100 Villeurbanne (FR); DELLOYE-BOURGEOIS, Céline, 69008 Lyon (FR)
(74) Mandataire: Regimbeau

(56) Documents cités:
- WO-A1-2016/005398
- CHRISTIAN BUSCH ET AL: "The Chick Embryo as an Experimental System for Melanoma Cell Invasion", PLOS ONE, vol. 8, no. 1, 14 January 2013 (2013-01-14), pages e53970, XP055714601, DOI: 10.1371/journal.pone.0053970
- TENE A CAGE ET AL: "Distinct patterns of human medulloblastoma dissemination in the developing chick embryo nervous system", CLINICAL & EXPERIMENTAL METASTASIS, KLUWER ACADEMIC PUBLISHERS, DO, vol. 29, no. 4, 10 February 2012 (2012-02-10), pages 371 - 380, XP035021844, ISSN: 1573-7276, DOI: 10.1007/S10585-012-9456-6
- CHRISTIAN BUSCH ET AL: "Human melanoma cells in the rhombencephalon of the chick embryo: a novel model for brain metastasis", EXPERIMENTAL DERMATOLOGY, BLACKWELL MUNSGAARD, COPENHAGEN; DK, vol. 21, no. 12, 22 November 2012 (2012-11-22), pages 944 - 947, XP071776857, ISSN: 0906-6705, DOI: 10.1111/EXD.12041
- ALEXANDRA CRETU ET AL: "Human and Rat Glioma Growth, Invasion, and Vascularization in a Novel Chick Embryo Brain Tumor Model", CLINICAL & EXPERIMENTAL METASTASIS, KLUWER ACADEMIC PUBLISHERS, DO, vol. 22, no. 3, 1 May 2005 (2005-05-01), pages 225 - 236, XP019235828, ISSN: 1573-7276, DOI: 10.1007/S10585-005-7889-X
- JARROSSON LORAINE ET AL: "An avian embryo patient-derived xenograft model for preclinical studies of human breast cancers", ISCIENCE, vol. 24, no. 12, 1 December 2021 (2021-12-01), US, pages 103423, XP093016810, ISSN: 2589-0042, DOI: 10.1016/j.isci.2021.103423
- KRISTIN H KAIN ET AL: "The chick embryo as an expanding experimental model for cancer and cardiovascular research", DEVELOPMENTAL DYNAMICS, WILEY-LISS INC NEW YORK , NY, US, vol. 243, no. 2, 19 December 2013 (2013-12-19), pages 216 - 228, XP071971335, ISSN: 1058-8388, DOI: 10.1002/DVDY.24093
- JAYACHANDRAN APARNA ET AL: "Embryonic Chicken Transplantation is a Promising Model for Studying the Invasive Behavior of Melanoma Cells", FRONTIERS IN ONCOLOGY, vol. 5, 16 February 2015 (2015-02-16), XP093016813, DOI: 10.3389/fonc.2015.00036

## Description

### DOMAINE DE L'INVENTION

La présente invention concerne un modèle d'étude animal des cellules cancéreuses, notamment issues de tumeurs solides humaines, et plus particulièrement des tumeurs cérébrales primaires et secondaires, des tumeurs pulmonaires et des tumeurs mammaires.

### INTRODUCTION

La modélisation des cancers humains chez l'animal de laboratoire est une problématique centrale dans le cadre des tests précliniques accompagnant le développement de nouvelles thérapies anti-cancéreuses. Les critères majeurs retenus pour les modèles animaux développés en ce sens sont la fiabilité du modèle, la rapidité d'exécution et le coût de réalisation.

Les modèles animaux développés pour l'étude des tumeurs et actuellement utilisés sont essentiellement des modèles murins. La préparation de ces modèles implique un temps de réalisation relativement long, et un coût important. De plus, certains types de cellules cancéreuses ne peuvent pas s'implanter dans des modèles animaux murins.

L'embryon de gallinacé, notamment de poulet ou de caille, est un modèle intéressant pour réaliser des expériences *ex vivo,* en particulier pour l'étude du développement embryonnaire et pour des expériences de xéno-transplantation. Il est en effet peu onéreux, très accessible et facile à manipuler. C'est un modèle de choix pour l'étude de la prolifération, de la différentiation et de la migration cellulaire. Ce modèle animal peut aussi être utilisé pour l'étude des tumeurs.

Un modèle classique d'étude sur l'embryon de gallinacé est la greffe de cellules exogènes au niveau des annexes de l'embryon, plus précisément sur la membrane chorio-allantoïdienne (CAM). Les cellules tumorales sont implantées sur la membrane de l'embryon de poulet. Après incubation d'environ 2 jours, une tumeur se forme. Cette tumeur profite du réseau vasculaire de la membrane de l'embryon, particulièrement développé, pour croître. Un tel système a permis de reproduire *in vivo* des tumeurs humaines, notamment de glioblastome, présentant des caractéristiques cellulaires et moléculaires semblables à celles observées dans les tumeurs *in vivo.* (Hagedorn *et al.,* 2005)

Ces greffes de cellules cancéreuses sur la membrane chorio-allantoïdienne ont notamment été utilisées pour déterminer le potentiel métastatique des cellules cancéreuses, les cellules greffées traversant la membrane étant jugées les plus susceptibles de métastaser (US 6,228,345).

Ce modèle de greffe sur membrane chorio-allantoïdienne est également largement utilisé pour cribler des molécules thérapeutiques, notamment des molécules destinées à inhiber l'angiogenèse tumorale (voir par exemple WO 2015/074050).

La demande de brevet US 2013/0171680 décrit la xénogreffe de cellules hématopoïétiques humaines malignes dans d'autres annexes embryonnaires : les cellules cancéreuses sont injectées dans le sac amniotique, dans le sac vitellin, ou dans les vaisseaux sanguins de la membrane CAM.

Jusqu'à présent, peu de travaux ont été réalisés sur des modèles d'embryon de gallinacé greffé avec des cellules cancéreuses au sein des tissus de l'embryon, et non dans ses annexes.

Carter et al. (Oncogenesis, 2012) ont injecté des cellules humaines de neuroblastome dans les vaisseaux sanguins d'un embryon de poulet, au stade de développement compris entre 3 et 6 jours. Au contact du micro-environnement embryonnaire, les cellules se re-programment en un phénotype plus bénin, notamment lorsqu'elles se fixent dans les tissus neuronaux.

Busch *et al.* (2013) décrivent l'injection de cellules de mélanome ou de cellules de cancer du sein dans l'embryon de poulet, et plus précisément : dans la lumière du tube neural ; dans la cupule optique ; ou dans le ventricule du cerveau postérieur (rhombencéphale). Il s'agit ici d'injections de cellules dans la lumière des vésicules du tube neural, et non d'une greffe au sein des tissus de l'embryon. Les cellules injectées s'agrègent en amas, mais ne migrent pas.

De même, Cage *et al.* (2012) ont étudié la dissémination leptoméningée de cellules de médulloblastome injectées à l'intérieur des ventricules cérébraux d'embryons de poulet. Les cellules injectées se dispersent en se mélangeant au liquide céphalorachidien, mais ne migrent pas au sein des tissus ni ne se reproduisent.

Kulesa et collaborateurs (PNAS, 2006) ont décrit la greffe de cellules humaines de mélanome, au niveau des crêtes neurales, dans des embryons de poulet à un stade de développement indiqué comme étant de « 6-8 somites » qui correspond à un stade compris entre HH8.5 et HH9.5 selon la nomenclature de référence. A ce stade de développement de l'embryon, les cellules transplantées ne forment pas de tumeurs, se re-programment en cellules bénignes et s'intègrent dans des tissus selon le profil de migration des mélanocytes.

Dans ces modèles, les cellules cancéreuses implantées ne sont pas capables de reproduire des tumeurs, *a fortiori* ne sont pas capables de reproduire des tumeurs dans des tissus homologues à ceux dont sont issues lesdites tumeurs.

Ainsi, bien que l'embryon de gallinacé soit un modèle animal de choix pour l'étude *ex vivo* des tumeurs humaines, les modèles développés jusqu'à maintenant ne permettent pas d'étudier la migration des cellules cancéreuses au sein d'un organisme vivant, ni d'étudier les tumeurs dans un micro-environnement tissulaire homologue à celui de la tumeur *in vivo.*

La présente invention est relative à la mise au point d'un modèle animal pour l'étude des cancers, notamment des cancers humains, dans lequel des cellules cancéreuses greffées au sein d'un embryon de gallinacé vont migrer et créer des foyers cancéreux dans des tissus de l'embryon correspondants aux tissus dont les cellules cancéreuses sont issues (greffes orthotopiques), ou dans d'autres tissus (greffes hétérotopiques).

### RESUME DE L'INVENTION

La présente invention est relative à un embryon de gallinacé, de poulet ou de caille, dans lequel ont été greffées, au sein des tissus de l'embryon, dans des sites spécifiques distincts de la lumière du tube neural, des cellules cancéreuses, lesdites cellules cancéreuses n'étant ni des cellules de neuroblastome ni des cellules de mélanome, et lesdites cellules se reproduisant et formant des tumeurs au sein de l'embryon, à un site d'implantation distinct du site de greffe .

La greffe est caractérisée en ce qu'elle est effectuée à un moment déterminé du développement de l'embryon, à savoir à un moment compris entre les stades HH10 et HH25, et plus spécifiquement entre les stades HH13 et HH15.

Un tel embryon greffé est un modèle animal d'étude des cancers, permettant de suivre la migration des cellules cancéreuses et le développement de tumeurs au sein de tissus homologues aux tissus dont proviennent les cellules cancéreuses, et/ou où ces cellules cancéreuses ont tendance à créer des foyers cancéreux secondaires, c'est-à-dire à métastaser.

Un tel embryon greffé est également un modèle animal d'étude des cancers qui peuvent s'implanter et/ou se développer dans différents tissus de l'embryon, de manière hétérotopique.

Ainsi, la présente invention concerne un embryon de gallinacé dans lequel des cellules cancéreuses ont été greffées au sein des tissus de l'embryon, lesdites cellules cancéreuses n'étant pas introduites dans la lumière du tube neural, caractérisé en ce que l'embryon est à un stade de développement compris entre le stade HH10 et le stade HH25 au moment de la greffe, et lesdites cellules cancéreuses n'étant ni des cellules de neuroblastome ni des cellules de mélanome, lesdites cellules se reproduisant et formant des tumeurs au sein de l'embryon, à un site d'implantation distinct du site de greffe, et ledit gallinacé étant un poulet (*gallus gallus*) ou une caille (*Coturnix japonica*)*.*

Autrement dit, la présente invention concerne un embryon de gallinacé comprenant au moins une tumeur composée de cellules cancéreuses qui ont été greffées au sein des tissus de l'embryon, caractérisé en ce que l'embryon est à un stade de développement compris entre le stade HH10 et le stade HH25 au moment de la greffe, lesdites cellules cancéreuses n'étant ni des cellules de neuroblastome ni des cellules de mélanome, ladite tumeur étant formée à un site d'implantation distinct du site de greffe, et ledit gallinacé étant un poulet (*gallus gallus*) ou une caille (*Coturnix japonica*)*.*

La présente invention concerne également un procédé de préparation d'un embryon de gallinacé dans lequel des cellules cancéreuses ont été greffées puis ont formé des tumeurs au sein dudit embryon, comprenant les étapes suivantes :
- greffe de cellules cancéreuses au sein des tissus dudit embryon, lesdites cellules cancéreuses n'étant pas introduites dans la lumière du tube neural, et
- incubation de l'embryon greffé pendant au moins 24 heures, lesdites cellules cancéreuses se reproduisant et formant des tumeurs au sein de l'embryon, dans un site d'implantation distinct du site de greffe ;
caractérisé en ce que :
ledit gallinacé est un poulet (*gallus gallus*) ou une caille (*Coturnix japonica*)*,*
l'embryon est à un stade de développement compris entre le stade HH10 et le stade HH25 au moment de la greffe, et
lesdites cellules cancéreuses n'étant ni des cellules de neuroblastome ni des cellules de mélanome.

La présente invention concerne également un procédé pour le suivi d'un patient présentant une tumeur, comprenant :
a) la préparation d'un premier embryon greffé selon le procédé décrit ci-dessus, avec des cellules cancéreuses issues dudit patient à un instant T₁, et l'appréciation de la tumorigenèse des tumeurs se développant dans ce premier embryon,
b) la préparation d'un second embryon greffé selon le procédé décrit ci-dessus, avec des cellules cancéreuses issues dudit patient à un instant T₂, et l'appréciation de la tumorigenèse des tumeurs se développant dans ce second embryon,
c) la comparaison entre la tumorigenèse des tumeurs se développant dans le premier embryon greffé et dans le second embryon greffé.

La présente invention est également relative à un procédé de criblage de molécules thérapeutiques destinées au traitement d'un cancer, consistant en les étapes suivantes :
a) préparation d'un embryon greffé selon le procédé décrit ci-dessus ;
b) administration à cet embryon greffé d'une molécule thérapeutique candidate;
c) appréciation de la malignité des cellules cancéreuses présentes dans cet embryon greffé après administration de ladite molécule candidate.

La présente invention est également relative à un procédé de préparation de tumeurs composées de cellules cancéreuses, comprenant les étapes suivantes :
i. préparation d'un embryon greffé selon le procédé décrit ci-dessus ;
ii. prélèvement desdites tumeurs formées au sein de l'embryon.

Ces tumeurs produites dans l'embryon peuvent être réutilisées comme le serait un prélèvement tumoral initial, par exemple pour la réalisation de cultures cellulaires, pour une implantation dans un autre modèle animal de cancer, ou pour des analyses de biochimie et/ou de biologie moléculaire sur lesdites tumeurs.

### LEGENDES DES FIGURES

**Figure 1****.** Représentation des premiers stades de développement de l'embryon de poulet, de HH12 (14 somites) à HH25 (52-54 somites).
**Figure 2****.** Tableau de correspondance des premiers stades de développement des embryons de caille (*Japanese quail*) et de poulet (*chick*) en fonction du temps écoulé depuis la fertilisation.

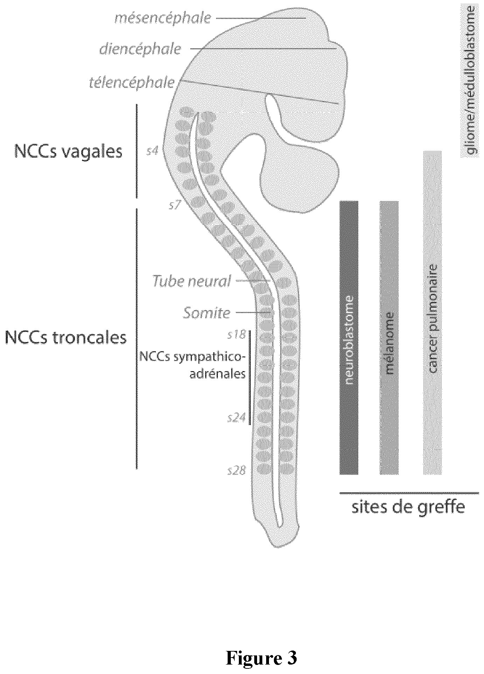

**Figure 3****.** Coupe longitudinale d'un embryon de poulet au stade 28 somites, soit au stade HH16. L'étendue des sites de greffe préférés, pour chaque type de cellules cancéreuses, est présentée à droite de la figure.
**Figure 4****.** Coupe transversale d'embryon greffé.
   Localisation des foyers cancéreux 48h après la greffe de cellules humaines de mélanome : les cellules greffées au niveau du toit dorsal du tube neural, entre les somites 18 à 24, forment des amas tumoraux au niveau sous-cutané ainsi que dans le mésenchyme bordant le tube neural (Cercles en pointillés).

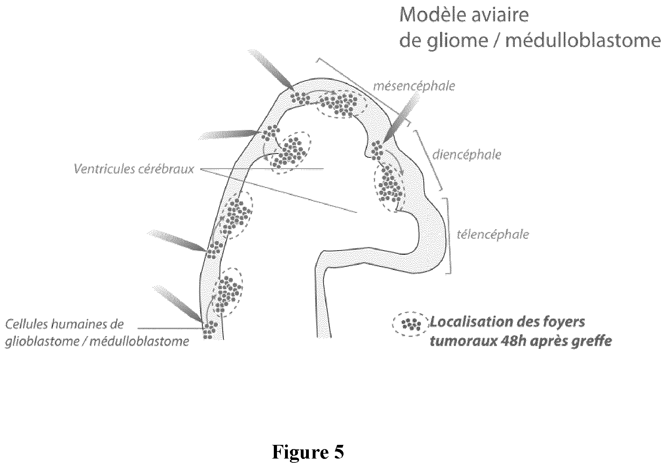

**Figure 5****.** Coupe longitudinale d'embryon greffé.
   Localisation des foyers cancéreux 48h après la greffe de cellules humaines de gliome ou de médulloblastome sur une zone allant de la crête neurale cervicale (en regard des somites 1 à 4) jusque dans les tissus bordant les ventricules cérébraux des différents territoires du cerveau : les cellules greffées forment des amas tumoraux dans les tissus cérébraux.

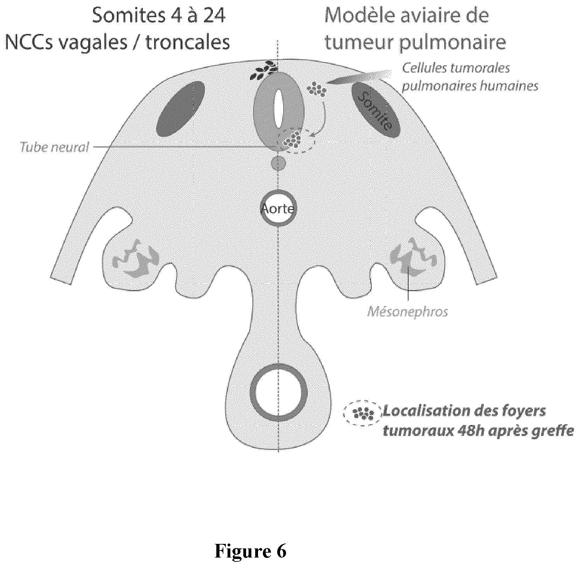

**Figure 6****.** Coupe transversale d'embryon greffé.
   Localisation des foyers cancéreux 48h après la greffe de cellules humaines de tumeur pulmonaire : les cellules greffées dans le mésenchyme latéral en regard des crêtes neurales vagales et troncales (somites 4 à 24) forment des amas tumoraux dans la corne ventrale du tube neural et le mésenchyme latéral. (Cercles en pointillés).

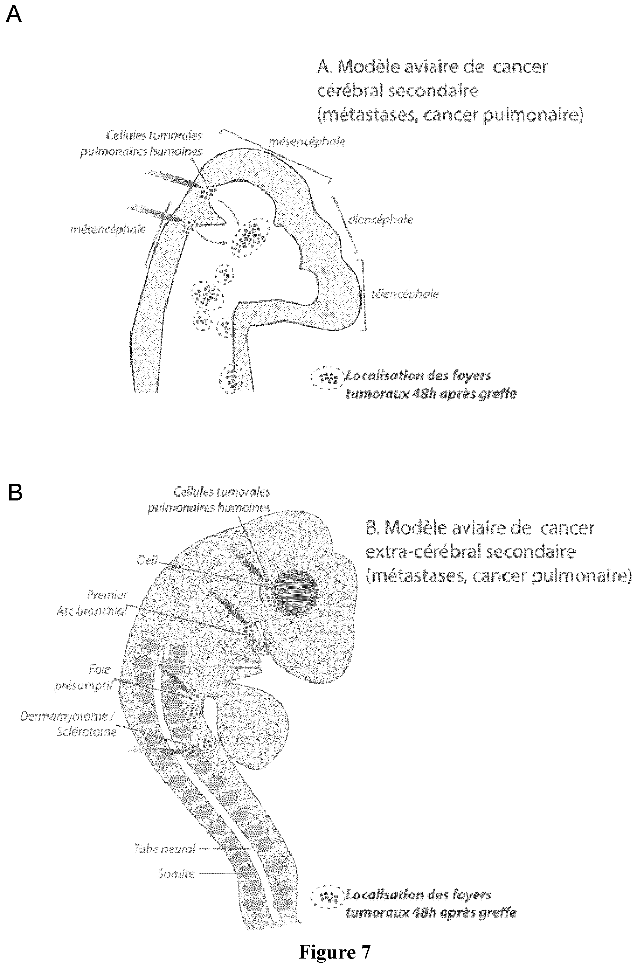

**Figure 7****.** Coupe longitudinale d'embryon greffé après la greffe de cellules humaines de cancer pulmonaire.
   A. Localisation cérébrale des foyers cancéreux 48h après la greffe de cellules humaines de cancer pulmonaire sur une zone allant de la crête neurale cervicale (en regard des somites 1 à 4) jusque dans les tissus bordant les ventricules cérébraux des différents territoires du cerveau : les cellules greffées forment des amas tumoraux dans les tissus cérébraux, à l'image des métastases pulmonaires cérébrales chez l'homme.
   **B.** Localisation extra-cérébrale des foyers cancéreux 48h après la greffe de cellules humaines de cancer pulmonaire dans des zones couvrant les sites présomptifs principaux de métastases des cancers pulmonaires humains (tissu périorbitaire, premier arc branchial, ébauche hépatique, ébauche des membres - sclérotome/dermamyotome): les cellules greffées forment des amas tumoraux dans le cartilage et les os de la face (greffes périorbitaire et dans le premier arc branchial), dans le foie embryonnaire (greffe dans l'ébauche hépatique) et dans les tissus dérivant des somites tels que le tissu osseux (greffe dans le sclérotome/dermamyotome).

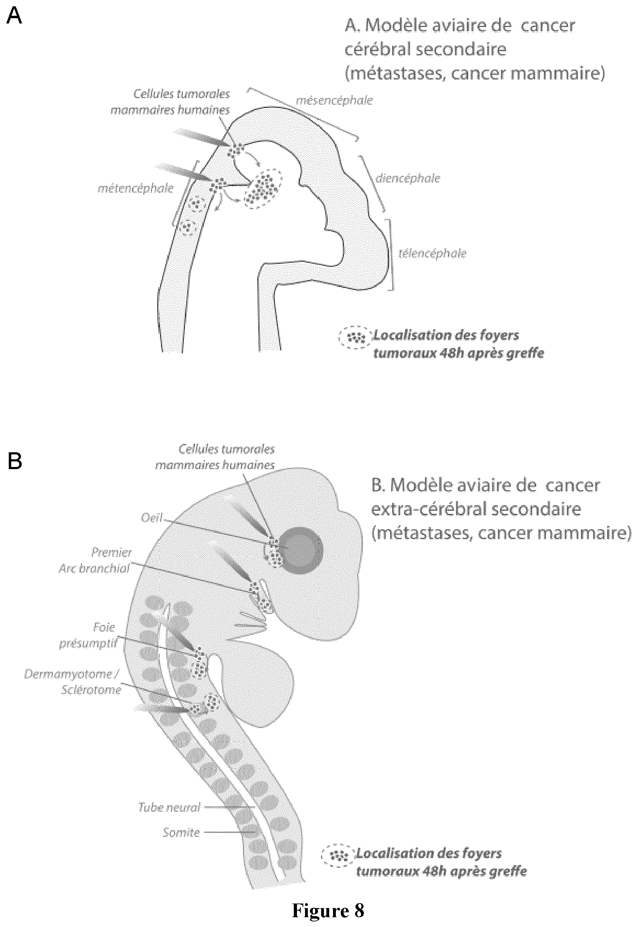

**Figure 8****.** Coupe longitudinale d'embryon greffé après la greffe de cellules humaines de cancer du sein.
   **A.** Localisation cérébrale des foyers cancéreux 48h après la greffe de cellules humaines de cancer du sein sur une zone allant de la crête neurale cervicale (en regard des somites 1 à 4) jusque dans les tissus bordant les ventricules cérébraux des différents territoires du cerveau : les cellules greffées forment des amas tumoraux dans les tissus cérébraux.
   **B.** Localisation extra-cérébrale des foyers cancéreux 48h après la greffe de cellules humaines de cancer du sein dans des zones couvrant les sites présomptifs principaux de métastases des cancers mammaires humains (tissu périorbitaire, premier arc branchial, ébauche hépatique, ébauche des membres - sclérotome/dermamyotome): les cellules greffées forment des amas tumoraux dans le cartilage et les os de la face (greffes périorbitaire et dans le premier arc branchial), dans le foie embryonnaire (greffe dans l'ébauche hépatique) et dans les tissus dérivant des somites tels que le tissu osseux (greffe dans le sclérotome/dermamyotome).

### DESCRIPTION DETAILLEE DE L'INVENTION

Les termes suivants sont définis pour une meilleure compréhension de l'invention.

Le terme « gallinacé » désigne un oiseau de l'ordre des galliformes (ou gallinacés) qui comprend les poulets, cailles, dindes, faisans, paons, pintades, et d'autres animaux de basse-cour. Dans la présente invention, l'embryon est issu d'un poulet (*gallus gallus*) ou d'une caille (*Coturnix japonica*)*,* deux espèces fréquemment utilisées en laboratoire.

Le terme « embryon de gallinacé » désigne un œuf de gallinacé fécondé et dans lequel un embryon se développe normalement, dans les conditions adéquates, notamment en étant placé dans un incubateur chauffé à une température comprise entre 37 °C et 39 °C. Le temps d'incubation nécessaire pour aboutir à l'éclosion de l'œuf est de 21 jours.

Dans le cadre de la présente invention, l'embryon « receveur » ou « récepteur » désigne un embryon de gallinacé avant l'étape de la greffe.

Dans le cadre de la présente invention, l'embryon « greffé » ou « dans lequel des cellules cancéreuses ont été greffées » désigne un embryon de gallinacé après l'étape de la greffe, et plus particulièrement désigne l'embryon greffé après au moins 24 heures d'incubation, dans lequel au moins une tumeur composée de cellules cancéreuses greffées s'est développée. Cet embryon greffé objet de l'invention est un modèle d'étude animal du cancer.

Par « cancer » on entend la pathologie caractérisée par la présence dans un organisme de cellules malignes formées à partir de la transformation, par mutations ou instabilité génétique, de cellules initialement normales de l'organisme atteint par cette pathologie.

Le terme « embryon greffé » ou « embryon dans lequel des cellules cancéreuses ont été greffées » désigne au sens de l'invention un « embryon chimérique » c'est à dire un embryon possédant des cellules issues d'au moins deux organismes différents : des cellules de gallinacé, et des cellules cancéreuses issues d'un autre organisme, devenant partie intégrante de l'embryon suite à leur acceptation comme greffon, et poursuivant leur développement en formant une ou des tumeurs solides et/ou en continuant à se développer selon un mode non régulé par les contrôles normaux de la division cellulaire, au sein des tissus de l'embryon de gallinacé receveur.

Il est entendu que l'embryon greffé est un embryon chimérique puisqu'il comprend deux types cellulaires issus de deux organismes différents ; cependant, il ne s'agit pas d'une « chimère » au sens propre, l'embryon n'étant pas destiné à se développer suffisamment de manière à créer un organisme adulte, mais servant uniquement de support aux cellules cancéreuses pendant un court laps de temps. En tout état de cause, il est entendu que cet embryon de gallinacé ne donnera pas naissance à un organisme vivant chimérique, mais sera détruit dès que l'étude du devenir des cellules cancéreuses greffées aura été complétée.

Au sens de l'invention, le terme « greffe » ou « transplantation » désigne l'introduction de cellules exogènes dans un organisme récepteur, au sein de tissus de l'embryon.

En particulier, ce terme ne désigne pas une introduction de cellules exogènes dans des annexes de l'embryon, telles que la membrane chorio-allantoïdienne, le sac vitellin ou le sac amniotique. De plus, ce terme ne désigne pas une injection de cellules dans la circulation sanguine de l'embryon.

La présente demande est en particulier relative à des xénogreffes, ce terme désignant le fait que les cellules introduites dans l'embryon receveur sont issues d'un organisme d'une espèce différente de celle de l'embryon receveur.

La greffe de cellules cancéreuses est réalisée dans des conditions appropriées permettant auxdites cellules de se reproduire, de migrer, et de former des tumeurs au sein de l'embryon receveur, à des sites d'implantation pertinents, soit en accord avec leur origine tissulaire soit dans des tissus différents de ceux habituellement colonisés par ce type de cellules cancéreuses, ledit site d'implantation étant distinct du site de greffe.

Ces « conditions appropriées » permettent la reproduction, au sein d'un modèle animal, de certains aspects de la maladie dénommée 'cancer', et notamment la formation de tumeurs.

Ces « conditions appropriées » sont basées sur le stade de développement de l'embryon receveur au moment où la greffe est réalisée, et sur le site de la greffe.

Le choix du site de greffe permet notamment de déterminer la migration des cellules cancéreuses et leur implantation dans différents tissus, pour y former des foyers cancéreux. Préférentiellement ces foyers cancéreux sont des tumeurs solides.

Ainsi, selon l'invention, les cellules cancéreuses greffées dans un tissu (site de greffe) vont migrer dans l'embryon greffé en fonction de ce site de greffe et s'implanter dans un second tissu distinct du site de greffe, ci-après dénommé « tissu d'implantation » ou « site d'implantation », pour former au moins une tumeur. Dans certains territoires, les cellules vont migrer très localement et s'implanter à proximité du site de greffe.

Plusieurs cas sont à considérer :
- il peut être souhaitable de pratiquer des greffes dites 'orthoptiques' correspondant à l'établissement de foyers cancéreux dans des tissus homologues à ceux dans lesquels les cellules cancéreuses considérées forment des foyers cancéreux primaires dans l'organisme dont elles sont issues. Ces foyers cancéreux peuvent résulter soit d'une greffe directe dans le territoire ciblé, soit d'une greffe dans une voie de migration conduisant les cellules dans ce territoire. Le choix de certains sites spécifiques de greffe permet d'orienter un tel adressage des cellules cancéreuses vers des tissus d'implantation, comme cela est exemplifié dans la présente demande ;
- il peut également être désiré de reproduire des foyers cancéreux dits secondaires, au sein de tissus dans lesquels les cellules cancéreuses ont tendance à métastaser ; là aussi, ces foyers cancéreux secondaires peuvent être obtenus en pratiquant soit une greffe directe dans le territoire ciblé, soit une greffe dans une voie de migration conduisant les cellules dans ce territoire, site d'implantation ;
- enfin, il peut être simplement souhaité de créer des foyers cancéreux s'implantant et se développant dans d'autres types de tissus que ceux d'origine des cellules cancéreuses. Ces greffes dites 'hétérotopiques' correspondent à l'implantation d'un foyer cancéreux dans un tissu qui est distinct de celui qui héberge les cellules cancéreuses dans l'organisme dont elles sont issues, qu'elles soient issues d'une tumeur primaire ou secondaire (issues d'une métastase).

Un tel modèle animal permet donc d'étudier à la fois la migration des cellules cancéreuses et leur implantation au sein de tissus spécifiques, ou d'étudier des foyers cancéreux formés au sein de tissus hétérologues.

Les principaux avantages de ce modèle animal sont, outre la spécificité des sites d'implantation des foyers cancéreux, son faible coût de revient et sa rapidité de préparation.

En outre, ce modèle animal permet d'initier le développement de certaines tumeurs qui pourront être prélevées puis transplantées dans un autre modèle animal, comme par exemple une souris, ou être regreffées dans l'embryon aviaire, ou encore servir pour la réalisation de cultures et de modèles *ex vivo* 3D, ou encore pour réaliser des analyses biochimiques et moléculaires.

### Stade de développement de l'embryon receveur

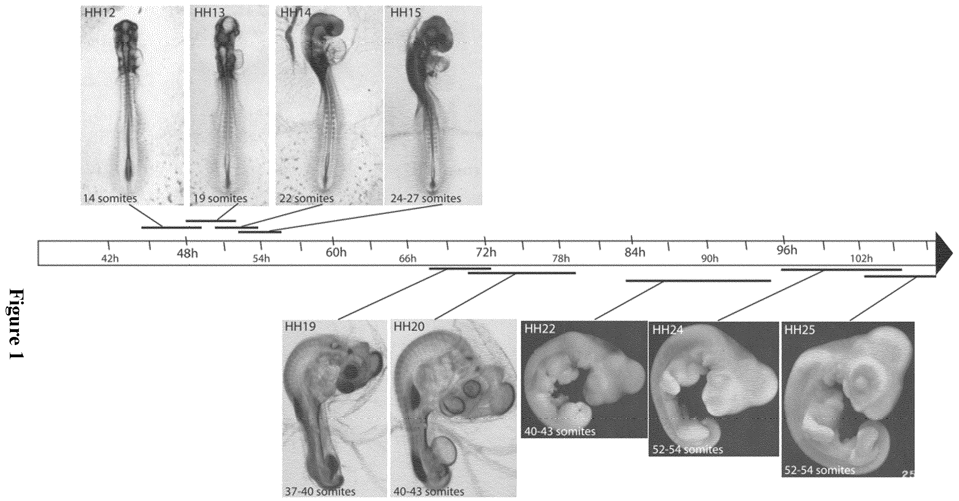

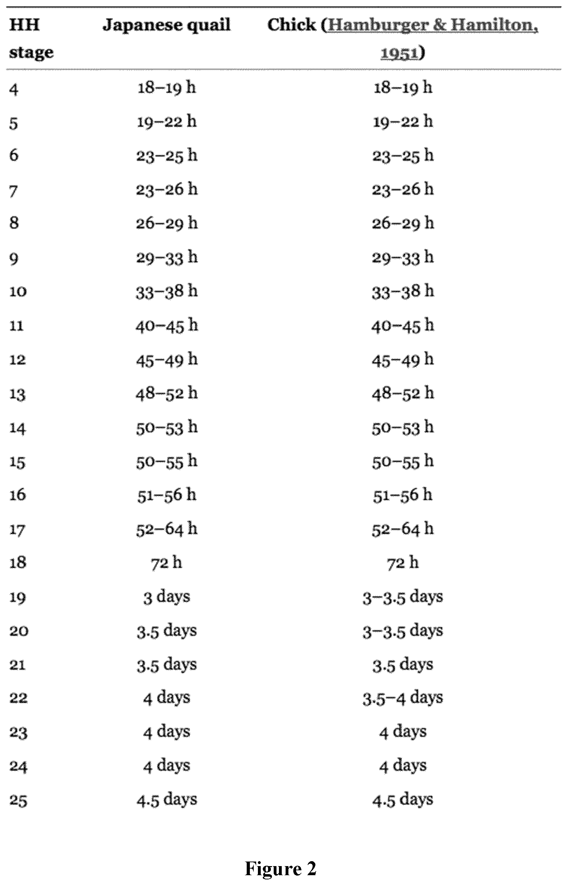

Plusieurs stades de développement de l'embryon de gallinacé ont été définis et sont représentés dans les figures 1 et 2. Ces stades ont été définis en fonction du temps d'incubation post-fécondation, et déterminé selon les critères définis par Hamburger et Hamilton (1951, J Morphol.). Par ailleurs, les somites apparaissant au fur et à mesure du développement, chaque stade est également caractérisé par un nombre de somites présentes.

Il est entendu que le développement de l'embryon ne commence que lorsque l'embryon est incubé dans de bonnes conditions, notamment à une température comprise entre 37°C et 39°C. Ainsi, un stade de développement « entre 48 et 55 heures » signifie que l'œuf a été maintenu cette durée dans les conditions optimales pour son développement. Un œuf fertilisé peut être maintenu à 14°C avant d'être placé dans des conditions optimales pour son développement ; cette période d'attente à 14°C n'est pas à prendre en compte dans la durée indiquée ci-dessous.

Selon l'invention, au moment de la greffe, l'embryon de poulet ou de caille est à un stade de développement compris entre le stade HH10 et le stade HH25.

Le stade HH10 est observé à environ 33 à 38 heures d'incubation, et est caractérisé par la présence de 10 somites.

Le stade HH25 est observé entre 102 et 108 heures d'incubation, et est caractérisé par la présence de 52 à 54 somites (voir figure 1).

Entre ces deux stades, un événement important est la courbure de l'embryon. En effet, à partir de l'apparition du 19ème somite (stade HH13), soit environ après 48 heures d'incubation post-fertilisation, la tête de l'embryon ébauche un mouvement de torsion lévogyre qui se propage pendant l'organogenèse, jusqu'à l'extrémité postérieure de l'embryon. La progression de ce mouvement est bien perceptible entre 55 et 68 heures d'incubation.

De préférence, au moment de la greffe, l'embryon de poulet ou de caille est à un stade de développement compris entre le stade HH12 et le stade HH25, soit à l'un des stades présentés en figure 1.

Cette phase du développement de l'embryon, prenant place entre 40h et 4.5 jours post-fécondation, est caractérisé par de nombreux évènements clefs de l'embryogenèse, parmi lesquels l'apparition des somites, la subdivision des grands territoires du cerveau, les courbures de différents domaines de l'embryon, et la formation de nombreux organes.

Selon un autre aspect préféré, au moment de la greffe, l'embryon de poulet ou de caille est à un stade de développement compris entre le stade HH10 et le stade HH18, entre le stade HH10 et le stade HH15 ou entre le stade HH12 et le stade HH16.

De préférence, la greffe de cellules cancéreuses est effectuée sur un embryon de gallinacé receveur à un stade de développement compris entre les stades HH13 et HH15 soit entre 48 et 55 heures post-fécondation, et de préférence entre 50 et 53 heures post-fécondation (stade HH14).

A ce stade de développement HH13-HH15, l'embryon de poulet ou de caille comprend entre 19 et 27 somites.

### Cellules cancéreuses

Au sens de l'invention, le terme « cellules cancéreuses » désigne des cellules malignes, c'est-à-dire capables de se diviser sans être soumises aux contrôles normaux de régulation de la division cellulaire, lesdites cellules cancéreuses n'étant nis des cellules de neuroblastome, ni des cellules de mélanome. La plupart des cellules cancéreuses présentent des caractères anormaux dits 'caractères cytologiques de malignité'.

Ces cellules peuvent former une ou des excroissances appelées dans la présente demande indifféremment 'tumeurs', 'néo-tumeurs', 'foyers tumoraux' 'foyers cancéreux', 'amas tumoraux' ou 'masses tumorales', se développant au sein d'un ou plusieurs tissus.

Par « tumeur » on désigne une prolifération cellulaire excessive aboutissant à une masse tissulaire, ayant tendance à persister et à croître, témoignant de son autonomie biologique. La présente invention concerne plus particulièrement les tumeurs malignes. Les tumeurs malignes ont habituellement une croissance rapide, et ont tendance à récidiver après éradication locale. Les tumeurs malignes sont mal limitées, non encapsulées ; leurs contours sont irréguliers.

Dans le cas des cellules cancéreuses circulantes, notamment sanguines, ces cellules sont caractérisées par une capacité de croissance et de division anarchique, non contrôlée.

Un organisme vivant présentant de telles cellules cancéreuses est diagnostiqué comme présentant un cancer.

Au sens de l'invention, les cellules cancéreuses destinées à être greffées dans les tissus d'un embryon receveur sont issues d'une tumeur maligne solide ou sont des cellules hématopoïétiques cancéreuses.

Selon un aspect préféré de l'invention, il s'agit de cellules cancéreuses issues de tumeurs malignes solides.

Il est entendu qu'au sens de l'invention, toutes les cellules cancéreuses sont concernées, à l'exception des cellules de neuroblastome et des cellules de mélanome.

Selon un aspect préféré de l'invention, les cellules cancéreuses greffées sont issues de tumeurs solides non pédiatriques.

Selon un aspect de l'invention, les cellules cancéreuses greffées sont issues de tumeurs malignes humaines se développant chez des individus adultes.

L'invention concerne de manière préférée un modèle animal destiné à étudier des tumeurs humaines, les cellules sont donc de manière préférée des cellules cancéreuses humaines. Il est néanmoins envisageable d'utiliser le modèle animal de l'invention pour l'étude de tumeurs animales non humaines, notamment pour l'étude de tumeurs se développant chez d'autres mammifères que l'être humain.

Selon un aspect de l'invention, les cellules cancéreuses greffées sont choisies parmi le groupe constitué de : des cellules issues de tumeurs cérébrales primaires ou secondaires, des cellules cancéreuses pulmonaires et des cellules de cancer du sein.

Il pourra également s'agir de cellules cancéreuses choisies parmi des cellules de tumeur mammaire HER2+/ER+, des cellules de cancer de la prostate, des cellules de sarcomes, des cellules de gliomes pédiatriques, et des cellules de cancer de poumon de type « EGFR muté ».

Selon l'invention, les cellules cancéreuses greffées ne sont pas des cellules de mélanome.

Selon encore un autre aspect de l'invention, les cellules cancéreuses greffées sont choisies parmi le groupe constitué de : des cellules issues de tumeurs cérébrales primaires ou secondaires, des cellules cancéreuses pulmonaires et des cellules de cancer du sein.

Pour la préparation de l'embryon de gallinacé greffé, les cellules cancéreuses peuvent être greffées sous la forme de :
- cellules en suspension, injectées dans les tissus cibles ;
- morceau solide (bloc) de tissu tumoral ;
- agrégat / homogénat de cellules cancéreuses isolées.

On entend par « greffon » dans la suite de la présente description un ensemble de cellules cancéreuses introduites sous forme groupée dans l'embryon receveur.

La greffe des cellules cancéreuses dans l'embryon de gallinacé receveur s'effectue selon les méthodes bien connues de l'homme du métier. L'embryon de gallinacé est en effet facilement accessible, après avoir réalisé une petite ouverture dans la coquille de l'œuf. En particulier, la greffe des cellules cancéreuses est réalisée à l'aide d'un micro-injecteur sous pression (Picopump PV830, World Precision Instruments). D'autres techniques de transplantation de cellules au sein de l'embryon de gallinacé ont été décrites dans l'art antérieur, par exemple par Kulesa et al. (PNAS, 2006) ou par Boulland et al. (JVE, 2010).

Selon un mode de réalisation préféré de l'invention, les cellules cancéreuses sont greffées en une quantité d'au moins environ 1000 cellules par greffon.

Alternativement, le greffon comprend une quantité d'au moins 5000, 10000, ou 15000 cellules cancéreuses par greffon.

Selon une autre alternative, le greffon comprendra une quantité de cellules cancéreuses allant d'environ 5000 à environ 75000 cellules par greffe, d'environ 10000 à environ 75000 cellules par greffe, ou d'environ 15000 cellules à environ 75000 cellules par greffe.

En particulier le greffon comprendra environ 15000, environ 20000, environ 25000, environ 30000, environ 35000, environ 40000, environ 45000, environ 50000, environ 55000, environ 60000, environ 65000, environ 70000, ou encore environ 75000 cellules cancéreuses.

La méthode permettant de comptabiliser les cellules est bien connue de l'homme du métier. En particulier, le nombre de cellules greffées avec le micro-injecteur est déterminé au préalable de la greffe en comptabilisant à l'aide d'une cellule de comptage de Malassez le nombre de cellules éjectées du capillaire, pendant un temps et à une pression donnée.

Selon un mode particulier de mise en œuvre de l'invention, plusieurs greffons comprenant chacun au moins 1000 cellules cancéreuses sont transplantés sur un seul embryon récepteur. En particulier, au moins deux, trois, quatre, cinq ou six greffons sont transplantés sur un seul embryon récepteur, en des sites appropriés.

Selon un mode préféré de mise en œuvre de l'invention les cellules cancéreuses greffées sont des cellules cancéreuses humaines.

Selon un mode particulier de mise en œuvre de l'invention les cellules cancéreuses greffées sont des cellules humaines issues d'une tumeur de patient, c'est-à-dire d'un individu humain atteint d'un cancer.

Les cellules cancéreuses ont été prélevées par des techniques bien connues de l'Homme du métier, telles que la biopsie et la micro-chirurgie.

Afin de distinguer les cellules cancéreuses greffées au sein de l'embryon de gallinacé, et notamment de suivre leur dispersion et leur capacité de multiplication, les cellules greffées sont avantageusement marquées par un colorant ou expriment une protéine marqueur.

Un tel marquage peut être réalisé à l'aide de colorants. Les cellules peuvent en particulier être marquées grâce à des colorants vitaux tels que des carbocyanides qui ont une affinité pour les membranes cellulaires, auxquelles ils s'incorporent, conférant aux cellules une fluorescence rouge. Les colorants de type succinimidyl esters de carboxyfluorescéine (CFSE), qui émettent une fluorescence verte lorsque qu'ils réagissent avec les protéines intracellulaires pourront également être utilisés.

Selon un aspect particulier de l'invention, les cellules cancéreuses greffées expriment une protéine marqueur.

Une protéine marqueur désigne une protéine codée par un gène exogène introduit dans la cellule par les méthodes classiques de génie génétique, l'expression de ce gène étant sous le contrôle d'un promoteur actif dans cette cellule, et cette protéine étant visible, ou étant capable de réagir avec un réactif chimique pour devenir visible. De nombreuses protéines marqueurs sont connues telles que la Green Fluorescent Protein (GFP).

### Incubation de l'embryon greffé

Suite à la greffe, l'embryon de gallinacé est incubé pendant au moins 24 heures selon une technique standard, dans un incubateur saturé en humidité, à une température comprise entre 37°C et 39 °C, et de manière préférée à environ 38,5 °C.

Dès 24 heures d'incubation, les premières tumeurs composées de cellules cancéreuses greffées sont observées au sein de l'embryon greffé.

Selon un aspect particulier de l'invention, l'embryon est incubé après la greffe de cellules cancéreuses pendant au moins 36 heures, au moins 48 heures, au moins 60 heures, au moins 72 heures, au moins 4 jours, au moins 5 jours, au moins 6 jours, au moins 7 jours, au moins 8 jours, et jusqu'à 20 jours dans le cas de l'étude de cellules cancéreuses migrant de façon lente et/ou ayant une cinétique plus longue pour la formation de tumeurs, au sein de l'embryon greffé.

Selon un aspect préféré de l'invention, l'embryon est incubé pendant environ 48 à 52 heures après la greffe, préférentiellement à une température comprise entre 37 C et 39 C.

### Site de la greffe

Selon un aspect préféré de l'invention, les cellules cancéreuses sont greffées dans l'embryon receveur au niveau du tube neural, entre les somites 1 à 24, et/ou dans les tissus cérébraux.

Le tube neural comprend le système nerveux primitif des embryons. Les somites désignent les structures embryonnaires situées de part et d'autre du tube neural et de la chorde, et sont composées d'unités répétées le long de l'axe antéro-postérieur de l'embryon. Au stade de développement compris entre 48 et 55 heures post-fécondation, c'est-à-dire entre les stades HH13 et HH15, l'embryon de gallinacé comprend de 19 à 27 somites. Une représentation de l'embryon de poulet à différents stades de développement, allant de 14 à 54 somites, est présentée en figure 1.

Au sens de l'invention, les expressions « dans le tube neural » ou « au niveau du tube neural » sont synonymes et signifient que les cellules cancéreuses sont introduites au sein des tissus constituant le tube neural, et non dans la lumière du tube neural (qui comprend les ventricules cérébraux et le canal central de la moelle épinière, dans lesquels circule le liquide céphalo-rachidien).

Selon un premier aspect de l'invention, les cellules cancéreuses sont greffées au sein des tissus constituant le tube neural, entre les somites 1 à 24.

Selon un deuxième aspect de l'invention, les cellules cancéreuses sont greffées dans les tissus cérébraux. On entend par « tissus cérébraux » les couches de tissus composés des neurones, les zones bordant les ventricules dans lesquels naissent les neurones, les plexi choroïdes et les membranes externes isolant le cerveau de l'extérieur comme la pie-mère et l'arachnoïde.

Les tissus cérébraux désignent les différents territoires du cerveau tels que : télencéphale, diencéphale, mésencéphale, mésenchyme cérébral et tronc cérébral.

Selon un troisième aspect de l'invention, les cellules cancéreuses sont greffées dans l'embryon receveur au sein des tissus constituant le tube neural, entre les somites 1 à 24, et sont également greffées dans les tissus cérébraux.

Selon la présente invention, le modèle animal constitué d'un embryon de gallinacé greffé avec des cellules cancéreuses est adapté pour l'étude de tout type de cellules cancéreuses. Le modèle animal est cependant principalement destiné à l'étude des tumeurs malignes solides humaines.

Il est rappelé qu'au sens de l'invention, le terme « cellules cancéreuses » désigne tout type de cellules cancéreuses à l'exception des cellules de neuroblastome et des cellules de mélanome, et inclut les cellules hématopoïétiques cancéreuses.

Selon encore un autre aspect de l'invention, les cellules cancéreuses sont choisies parmi le groupe constitué de : des cellules issues de tumeurs cérébrales primaires ou secondaires, des cellules cancéreuses pulmonaires et des cellules de cancer du sein.

Pour chaque type de cellules cancéreuses, l'Homme du métier est en mesure de déterminer le site de greffe optimal, afin d'orienter la migration des cellules cancéreuses vers un lieu de développement de tumeur soit en adéquation avec le type cellulaire concerné, soit afin d'obtenir une néo-tumeur hétérotopique, se développant dans des tissus différents de ceux d'origine des cellules cancéreuses.

En particulier, les cellules cancéreuses peuvent être greffées en certains sites bien définis, afin d'être « adressées » spécifiquement dans certains tissus de l'embryon, où elles s'implanteront et formeront des tumeurs dans des tissus équivalents aux tissus d'où elles proviennent, ou dans des tissus où des tumeurs secondaires métastatiques ont tendance à apparaitre.

Selon l'invention, les cellules cancéreuses sont greffées dans un premier tissu distinct du tissu d'implantation où se forme la ou les tumeurs, la greffe dans le premier tissu orientant les cellules cancéreuses greffées vers le tissu d'implantation où elles constituent la ou les tumeurs, autrement dit là où les tumeurs s'établissent.

Plusieurs types cellulaires ont été greffés dans un embryon de gallinacé receveur, selon le procédé décrit dans la présente demande, et notamment des cellules cancéreuses choisies parmi le groupe constitué de : des cellules issues de tumeurs cérébrales primaires ou secondaires, des cellules cancéreuses pulmonaires et des cellules de cancer du sein.

### Exemple hors invention :

Des cellules de mélanomeont été greffées dans le toit dorsal du tube neural ou dans sa proximité latérale, entre les somites 18 à 24.

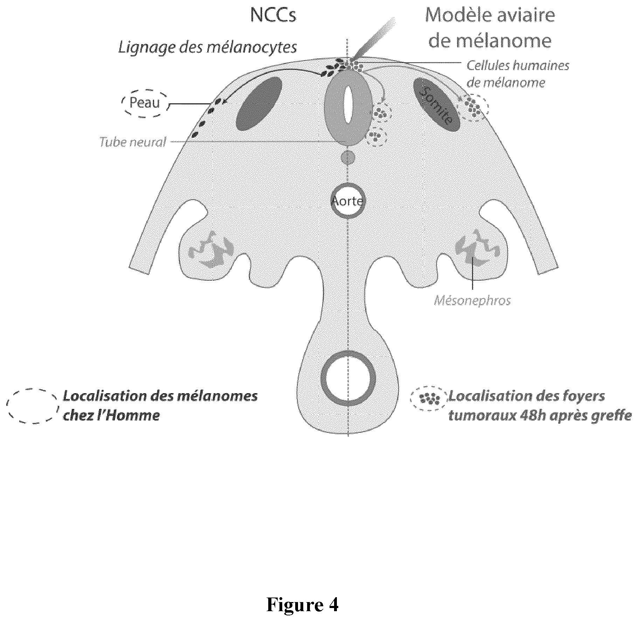

Comme représenté en figure 4, cet emplacement spécifique de la greffe permet d'obtenir, après au moins 24 heures d'incubation, un embryon greffé où des tumeurs composées de cellules transplantées migrent puis se développent spécifiquement sous la peau, reproduisant ainsi l'environnement tissulaire des cellules de mélanome lorsque celles-ci sont dans leur organisme initial.

Ainsi, les cellules cancéreuses greffées migrent au sein de l'embryon receveur afin de former des néo-tumeurs dans les tissus équivalents aux tissus humains dont elles sont issues.

### Exemples selon l'invention :

1) Des cellules cancéreuses issues de tumeurs cérébrales primaires ou secondaires sont greffées dans le tube neural entre les somites 1 à 4, et/ou dans les tissus cérébraux.

Dans cette mise en œuvre particulière, les cellules cancéreuses issues de tumeurs cérébrales primaires ou secondaires sont greffées dans le tube neural entre les somites 1 à 4, ou au niveau des tissus cérébraux, dans l'épaisseur du tissu cérébral ou en bordure des ventricules cérébraux.

On entend par « tissus cérébraux » les couches de tissus composés des neurones, les zones bordant les ventricules dans lesquels naissent les neurones, les plexi choroïdes et les membranes externes isolant le cerveau de l'extérieur comme la pie-mère et l'arachnoïde.

Dans une mise en œuvre particulière, au moins deux greffons de cellules cancéreuses issues de tumeurs cérébrales primaires ou secondaires sont greffés l'un dans le tube neural entre les somites 1 à 4, et l'autre au niveau des tissus cérébraux.

Le terme « tumeurs cérébrales primaires » désigne notamment des tumeurs telles que celles observées dans le gliome, le glioblastome ou le médulloblastome.

Par « tumeur cérébrale secondaire », on entend une tumeur formée dans le cerveau, suite à la dissémination de cellules cancéreuses métastatiques issues d'une tumeur dite 'primaire'. Cette tumeur primaire peut se trouver dans différents organes. Les cancers suivants sont ceux qui se propagent le plus souvent au cerveau : poumon, sein, mélanome, rein, testicule, colorectal, bronches, lymphome (surtout le lymphome non hodgkinien) et leucémie.

Comme représenté en figure 5, ces deux emplacements spécifiques de la greffe permettent d'obtenir, après au moins 24 heures d'incubation, un embryon greffé où des tumeurs composées de cellules transplantées se développent spécifiquement dans les tissus cérébraux, reproduisant ainsi l'environnement tissulaire des cellules de gliome, glioblastome ou de médulloblastome, lorsque celles-ci sont dans leur organisme initial.

2) Des cellules cancéreuses issues de tumeurs pulmonaires sont greffées dans le tube neural entre les somites 4 à 24.

Comme représenté en figures 6 et 7A, cet emplacement spécifique de la greffe permet d'obtenir, après au moins 24 heures d'incubation, un embryon greffé où des tumeurs composées de cellules transplantées se développent spécifiquement dans la corne ventrale du tube neural et le mésenchyme attenant. Ce site de formation, qui correspond à une région du système nerveux central, est une alternative au site de formation des métastases cérébrales. Il est donc représentatif de l'implantation d'une tumeur cancéreuse secondaire dans le système nerveux.

3) Des cellules cancéreuses issues de tumeurs mammaires (de cancer du sein) sont greffées dans le tube neural entre les somites 4 à 24.

Comme représenté en figure 7B, cet emplacement spécifique de la greffe permet d'obtenir, après au moins 24 heures d'incubation, un embryon greffé où des tumeurs composées de cellules transplantées se développent spécifiquement dans les tissus cérébraux, notamment à proximité de la couche cutanée.

### Procédés

La présente invention concerne également un procédé de préparation d'un embryon de gallinacé dans lequel des cellules cancéreuses ont été greffées puis ont formé des tumeurs au sein dudit embryon, comprenant les étapes suivantes :
- greffe de cellules cancéreuses au sein des tissus d'un embryon de gallinacé, lesdites cellules cancéreuses n'étant pas introduites dans la lumière du tube neural ; et
- incubation de l'embryon greffé pendant au moins 24 heures, lesdites cellules cancéreuses se reproduisant et formant des tumeurs au sein de l'embryon, dans un site d'implantation distinct du site de greffe ;
caractérisé en ce que ledit gallinacé est un poulet (*gallus gallus*) ou une caille (*Coturnix japonica*)*,* l'embryon est à un stade de développement compris entre le stade HH10 et le stade HH25 au moment de la greffe, et lesdites cellules cancéreuses n'étant ni des cellules de neuroblastome ni des cellules de mélanome.

Avantageusement, la greffe est réalisée dans le tube neural entre les somites 1 à 24 et/ou dans les tissus cérébraux.

Avantageusement, l'embryon greffé est incubé pendant environ 48 à 52 heures après la greffe, à une température comprise entre 37 °C et 39 °C.

Avantageusement, les cellules cancéreuses sont issues d'une tumeur prélevée sur un patient, et sont greffées en une quantité d'au moins 1000 cellules / greffon.

Avantageusement, la greffe est effectuée selon les conditions particulières détaillées précédemment.

La présente invention concerne également un procédé de suivi d'un patient présentant un cancer, comprenant :
a) la préparation d'un premier embryon greffé selon le procédé précédemment décrit, avec des cellules cancéreuses issues dudit patient à un instant T₁, et l'appréciation de l'indice de malignité des cellules cancéreuses se développant dans ce premier embryon,
b) la préparation d'un second embryon greffé selon le procédé précédemment décrit, avec des cellules cancéreuses issues dudit patient à un instant T₂, et l'appréciation de l'indice de malignité des cellules cancéreuses se développant dans ce second embryon,
c) la comparaison entre l'indice de malignité des cellules cancéreuses se développant dans le premier embryon et dans le second embryon.

« L'appréciation de l'indice de malignité» est effectuée par plusieurs approches complémentaires ; après prélèvement des cellules cancéreuses se développant dans l'embryon greffé, celles-ci sont soumises à différentes analyses :
- des études biochimiques et transcriptomiques, et
- des études *in vitro* via leur remise en culture.

Ces différentes analyses permettent notamment de déterminer l'indice de malignité relatif aux facteurs suivants :
- Détermination de l'index prolifératif des cellules cancéreuses par détection du marqueur Ki67 ;
- Détermination de l'index de mort cellulaire des cellules par détection des événements de mort cellulaire (fragmentation de l'ADN, nécrose, relargage du cytochrome c, activation des protéases pro-apoptotiques)
- Analyse du transcriptome et du protéome des cellules ;
- Etude du comportement cellulaire après remise en culture.

L'analyse combinée de tous ces facteurs, bien connus de l'homme du métier, permet de déterminer un 'indice de malignité' qui permet de quantifier la gravité et l'agressivité du cancer. En effet, l'ensemble de ces paramètres permet d'évaluer l'état de différenciation des cellules cancéreuses ainsi que leur capacité à proliférer et à se disséminer dans l'organisme. Ces paramètres font partie intégrante de la classification anatomo-pathologique des cellules cancéreuses sur lesquels s'appuient les cliniciens pour orienter la prise en charge thérapeutique.

La présente invention concerne également un procédé de suivi d'un patient présentant une tumeur, notamment une tumeur maligne solide, comprenant :
a) la préparation d'un premier embryon greffé selon le procédé précédemment décrit, avec des cellules cancéreuses issues dudit patient à un instant T₁, et l'appréciation de la tumorigenèse des tumeurs se développant dans ce premier embryon,
b) la préparation d'un second embryon greffé selon le procédé précédemment décrit, avec des cellules cancéreuses issues dudit patient à un instant T₂, et l'appréciation de la tumorigenèse des tumeurs se développant dans ce second embryon,
c) la comparaison entre la tumorigenèse des tumeurs se développant dans le premier embryon et dans le second embryon.

Par « patient présentant une tumeur », on entend un être humain affecté d'un cancer et présentant une tumeur solide sur un organe donné.

« L'appréciation de la tumorigenèse des tumeurs » est effectuée par plusieurs approches complémentaires ; après prélèvement des tumeurs apparues dans l'embryon greffé par microdissection, celles-ci sont soumises à différentes analyses :
- des études biochimiques et transcriptomiques, et
- des études *in vitro* via leur remise en culture.

Ces différentes analyses permettent notamment de déterminer les « facteurs de tumorigenèse » suivants :
- Localisation des foyers tumoraux par analyse histologique ;
- Mesure du volume tumoral à partir d'images reconstruites en 3 dimensions ;
- Détermination de l'index prolifératif au sein des foyers tumoraux par détection du marqueur Ki67 ;
- Détermination de l'index de vascularisation des foyers tumoraux par détection de marqueurs d'angiogenèse ;
- Détermination de l'index de mort cellulaire au sein des foyers tumoraux par détection des événements de mort cellulaire (fragmentation de l'ADN, nécrose, relargage du cytochrome c, activation des protéases pro-apoptotiques)
- Analyse du transcriptome et du protéome des tumeurs extraites *in situ* ;
- Etude du comportement cellulaire après remise en culture.

L'analyse combinée de tous ces facteurs, bien connus de l'homme du métier, permet de déterminer un 'indice de tumorigenèse' qui permet de quantifier la gravité et l'agressivité de la tumeur. En effet, l'ensemble de ces paramètres permet d'évaluer l'état de différenciation des cellules cancéreuses ainsi que leur capacité à proliférer et à se disséminer dans l'organisme. Ces paramètres font partie intégrante de la classification anatomo-pathologique des tumeurs sur lesquels s'appuient les cliniciens pour orienter la prise en charge thérapeutique.

Ainsi, il est possible de distinguer :
- des tumeurs se développant suite à la greffe, dans un embryon de gallinacé, de cellules cancéreuses issues d'un patient à un instant T1, et
- des tumeurs se développant suite à la greffe, dans un embryon de gallinacé, de cellules cancéreuses issues d'un patient à un instant T2.

Un tel procédé permet de suivre de manière *ex vivo* le développement de la tumeur solide d'un patient, et notamment l'indice de tumorigenèse de ses cellules cancéreuses à un instant T₀ (par exemple, avant le début d'un traitement) et à un instant T1, T2, T3 (par exemple, quelques mois après le début du traitement du patient).

Le procédé peut naturellement être répété le nombre de fois nécessaire pour suivre l'évolution de la tumeur chez un patient donné.

La présente invention concerne également un procédé de criblage de molécules thérapeutiques destinées au traitement d'un cancer, consistant en les étapes suivantes :
a) préparation d'un embryon greffé selon le procédé précédemment décrit;
b) administration à cet embryon d'une molécule thérapeutique candidate;
c) appréciation de la malignité des cellules cancéreuses présentes dans cet embryon après administration de ladite molécule candidate.

Par « molécule thérapeutique candidate » on entend une molécule chimique ou biologique potentiellement efficace pour traiter le cancer concerné.

L'étape b) peut être réalisée de plusieurs manières : la molécule peut être administrée à l'embryon avant ou après que la greffe de cellules cancéreuses ait été effectuée. En particulier, la molécule thérapeutique peut être injectée dans le réseau vasculaire de l'embryon, peut être incorporée dans le sac vitellin, ou peut être utilisée sur le greffon avant ou au moment de la réalisation de la greffe.

Selon cet aspect de l'invention, les cellules cancéreuses destinées à être greffées sur l'embryon receveur sont incubées avec une molécule thérapeutique avant/pendant la greffe sur l'embryon receveur.

L'appréciation de la malignité des cellules cancéreuses est effectuée par les approches décrites ci-dessus, après prélèvement des cellules cancéreuses se développant dans l'embryon greffé. La comparaison de la malignité des cellules cancéreuses au temps T0 et des cellules cancéreuses après au moins 24 heures, et en particulier après 1 (T1), 2 (T2) ou 3 (T3) jours d'administration de la molécule testée, permet de déterminer l'effet de la molécule thérapeutique administrée.

Naturellement l'administration de cette molécule peut être réalisée pendant différentes durées, notamment pendant au moins 24h, 48 h, 72 h, 96 h, et jusqu'à 5 jours, 6 jours, 7 jours, 8 jours, 9 jours, 10 jours, 11 jours, 12 jours, 13 jours, 14 jours, 15 jours, 16 jours, 17 jours, 18 jours, 19 jours, 20 jours, 21 jours soit jusqu'à l'éclosion de l'œuf, sous réserve que les tumeurs soient toujours présentes dans l'embryon de gallinacé.

Il est entendu qu'après les différents tests effectués, l'embryon est sacrifié selon les règles éthiques en vigueur.

La présente invention concerne également l'utilisation d'un embryon de gallinacé selon l'invention, pour permettre le développement de tumeurs composées de cellules cancéreuses.

Notamment, il est possible d'obtenir le développement *in vivo* de tumeurs composées de cellules cancéreuses dans l'embryon de gallinacé selon l'invention, alors que ces cellules cancéreuses ont des difficultés pour s'implanter et former des tumeurs dans des modèles animaux mammifères, tels que par exemple dans des souris.

En particulier, il a été observé que les cellules cancéreuses suivantes s'implantent difficilement, après un greffe dans des tissus d'animaux mammifères : les cellules de cancer du foie, les cellules de cancer de la prostate, et les cellules issues de cancers peu prolifératifs telles que les cellules issues des tumeurs mammaires HER2+/ER+, les cellules de sarcomes et les cellules de tumeurs cérébrales pédiatriques.

Le présent modèle animal permet avantageusement le développement de tumeurs composées de cellules cancéreuses, lesdites cellules cancéreuses ayant généralement des difficultés pour s'implanter après avoir été greffées dans un modèle animal mammifère.

La présente invention concerne donc un procédé de préparation de tumeurs composées de cellules cancéreuses, comprenant les étapes suivantes :
- préparation d'un embryon greffé selon le procédé précédemment décrit ;
   et
- prélèvement desdites tumeurs formées au sein de l'embryon.

Les tumeurs ainsi prélevées pourront ensuite être utilisées comme le serait un prélèvement tumoral initial, par exemple pour la réalisation de cultures cellulaires, pour être implantées dans un autre modèle animal, ou pour effectuer des analyses biochimiques et/ou de biologie moléculaire desdites tumeurs.

Selon l'invention, les cellules cancéreuses greffées ne sont pas des cellules de neuroblastome, ni des cellules de mélanome.

Selon encore une autre mise en œuvre du procédé de l'invention, les cellules cancéreuses greffées sont choisies parmi le groupe constitué de : des cellules issues de tumeurs cérébrales primaires ou secondaires, des cellules cancéreuses pulmonaires et des cellules de cancer du sein.

Selon encore une autre mise en œuvre du procédé de l'invention, les cellules cancéreuses greffées sont choisies parmi : des cellules de tumeur mammaire HER2+/ER+, des cellules de cancer de la prostate, des cellules de sarcomes, des cellules de gliomes pédiatriques, et des cellules de cancer de poumon de type « EGFR muté ».

Comme préalablement indiqué, il est entendu qu'après la réalisation des différents procédés selon l'invention, l'embryon de gallinacé est sacrifié selon les règles éthiques en vigueur.

La présente description inclut également les objets suivants :
∘ Un embryon de gallinacé dans lequel des cellules cancéreuses ont été greffées au sein des tissus de l'embryon, caractérisé en ce que l'embryon est à un stade de développement compris entre le stade HH10 et le stade HH25 au moment de la greffe, lesdites cellules cancéreuses n'étant pas des cellules de neuroblastome, et lesdites cellules formant des tumeurs au sein de l'embryon.
∘ Un embryon de gallinacé tel que décrit ci-dessus, caractérisé en ce que l'embryon est à un stade de développement compris entre les stades HH13 et HH15 au moment de la greffe.
∘ Un embryon de gallinacé tel que décrit ci-dessus, caractérisé en ce que l'embryon est incubé pendant au moins 24 heures après la greffe.
∘ Un embryon de gallinacé tel que décrit ci-dessus, caractérisé en ce que les cellules cancéreuses sont greffées en une quantité d'au moins 1000 cellules par greffe.
∘ Un embryon de gallinacé tel que décrit ci-dessus, caractérisé en ce que les cellules cancéreuses greffées sont des cellules humaines issues d'une tumeur de patient.
∘ Un embryon de gallinacé tel que décrit ci-dessus, caractérisé en ce que les cellules cancéreuses greffées sont marquées par un colorant ou expriment une protéine marqueur.
∘ Un embryon de gallinacé tel que décrit ci-dessus, caractérisé en ce que les cellules cancéreuses sont greffées dans un premier tissu distinct du tissu d'implantation où se forment les tumeurs, la greffe dans le premier tissu orientant les cellules cancéreuses greffées vers le tissu d'implantation où les tumeurs s'établissent.
∘ Un embryon de gallinacé tel que décrit ci-dessus, caractérisé en ce que les cellules cancéreuses sont greffées dans le tube neural entre les somites 1 à 24 et/ou dans les tissus cérébraux.
∘ Un embryon de gallinacé tel que décrit ci-dessus, caractérisé en ce que les cellules cancéreuses sont greffées dans les tissus cérébraux.
∘ Un embryon de gallinacé tel que décrit ci-dessus, caractérisé en ce que les cellules cancéreuses greffées sont choisies parmi le groupe constitué de : des cellules de mélanome, des cellules issues de tumeurs cérébrales primaires ou secondaires, des cellules cancéreuses pulmonaires et des cellules de cancer du sein.
∘ Un embryon de gallinacé tel que décrit ci-dessus, caractérisé en ce que les cellules cancéreuses greffées sont des cellules de mélanome, et sont greffées dans le toit dorsal du tube neural ou dans sa proximité latérale, entre les somites 18 à 24.
∘ Un embryon de gallinacé tel que décrit ci-dessus, caractérisé en ce que les cellules cancéreuses greffées sont issues de tumeurs cérébrales primaires ou secondaires, et sont greffées dans le tube neural entre les somites 1 à 4, et/ou dans les tissus cérébraux.
∘ Un procédé de préparation d'un embryon de gallinacé dans lequel des cellules cancéreuses ont été greffées puis ont formé des tumeurs au sein dudit embryon, comprenant les étapes suivantes :
   - greffe de cellules cancéreuses au sein des tissus d'un embryon de gallinacé, et
   - incubation de l'embryon greffé pendant au moins 24 heures, caractérisé en ce que l'embryon est à un stade de développement compris entre le stade HH10 et le stade HH25 au moment de la greffe, et lesdites cellules cancéreuses n'étant pas des cellules de neuroblastome.
∘ Un procédé de suivi d'un patient présentant une tumeur, comprenant :
   - la préparation d'un premier embryon greffé selon le procédé tel que décrit ci-dessus, avec des cellules cancéreuses issues dudit patient à un instant T1, et l'appréciation de la tumorigenèse des tumeurs se développant dans ce premier embryon,
   - la préparation d'un second embryon greffé selon le procédé tel que décrit ci-dessus, avec des cellules cancéreuses issues dudit patient à un instant T2, et l'appréciation de la tumorigenèse des tumeurs se développant dans ce second embryon,
   - la comparaison entre la tumorigenèse des tumeurs se développant dans le premier embryon greffé et dans le second embryon greffé.
∘ Un procédé de criblage de molécules thérapeutiques destinées au traitement d'un cancer, consistant en les étapes suivantes :
   - préparation d'un embryon greffé selon le procédé tel que décrit ci-dessus ;
   - administration à cet embryon greffé d'une molécule thérapeutique candidate;
   - appréciation de la malignité des cellules cancéreuses présentes dans cet embryon greffé après administration de ladite molécule candidate.
∘ Un procédé de préparation de tumeurs composées de cellules cancéreuses, comprenant les étapes suivantes :
   - greffe de cellules cancéreuses au sein des tissus d'un embryon de gallinacé à un stade de développement compris entre le stade HH10 et le stade HH25 au moment de la greffe, lesdites cellules cancéreuses n'étant pas des cellules de neuroblastome,
   - incubation de l'embryon greffé pendant au moins 24 heures, et
   - prélèvement desdites tumeurs formées au sein de l'embryon.

### EXEMPLES

Les exemples ci-dessous ont pour seule vocation d'illustrer l'invention, et en aucun cas ne limite l'invention aux mises en œuvre particulières décrites ci-dessous.

### MATERIEL ET METHODES

### Lignées cellulaires cancéreuses humaines

Des cellules cancéreuses humaines pulmonaires (lignée A549), de mélanome (lignée A375P), de glioblastome (lignée U251) de médulloblastome (lignée DEV) et de cancer du sein (lignée MDA MB 436) ont été manipulées génétiquement pour exprimer de façon stable la protéine fluorescente GFP.

### Embryons de poulet

Les œufs de poulet (*Gallus gallus*) fertilisés ont été achetés auprès d'un fournisseur (EARL Morizeau, Dangers, France) et maintenus à 14 °C jusqu'à utilisation. Les œufs ont été incubés à 38,5 °C pendant 52 heures dans un incubateur au taux d'humidité saturé, de façon à obtenir des embryons au stade de développement HH14.

### Greffes de lignées cancéreuses humaines dans l'embryon de poulet

5x10⁶ cellules cancéreuses ont été récoltées et re-suspendues dans 30 µL de milieu.

Après 52 heures d'incubation à 38,5 C, une fenêtre a été découpée dans la coquille afin de visualiser et d'accéder à l'embryon. La membrane vitelline a été découpée au niveau du tube neural et une blessure a été réalisée au niveau du toit du tube neural, face aux somites 20 et 21.

La suspension de cellules a été insérée dans un micro-capillaire en verre et des cellules déposées à l'aide d'un micro-injecteur sous pression dans chaque embryon (Picopump PV830, World Precision Instruments).

Les œufs ont ensuite été replacés dans l'incubateur à 38,5 C pendant 48 heures.

### Autres conditions

Plusieurs quantités de cellules cancéreuses greffées dans un embryon ont été testées pour réaliser la greffe : 1000 cellules, 3000 cellules, 10.000 (10⁴) cellules et 5.10⁶ cellules.

Différents stades de développement de l'embryon receveur ont également été testés pour le moment de la greffe : stades HH10 (10 somites), HH11 (13 somites) et HH14 (22 somites).

### Coupes d'embryons de poulet

Les embryons ont été récoltés et fixés en paraformaldéhyde 4 %, sur la nuit à 4 C. Selon le type d'analyse souhaitée, les embryons ont été coupés pour la réalisation de coupes transversales et sagittales longitudinales. Les coupes ont été maintenues en PBS à 4 C à l'abri de la lumière jusqu'à utilisation. La localisation des tumeurs a été étudiée par différents marquages et/ou détection de la fluorescence des cellules cancéreuses préalablement transformées pour exprimer la GFP (green fluorescent protein).

Le marquage consiste en l'incubation des cellules dans un colorant fluorescent vital, le CFSE, préalablement à la greffe. Plusieurs concentrations de ce colorant vital ont été testées, permettant d'optimiser la détection des masses tumorales formées dans l'embryon après la greffe.

### Prélèvement des tumeurs in situ et analyse

Les tumeurs sont prélevées par microdissection et soumises à différentes analyses :
- des études biochimiques et transcriptomiques (caractérisation et recherche de marqueurs moléculaires connus ou nouveaux), et
- des études *in vitro* via leur remise en culture.

### Prise d'images et traitement

Les coupes ont été analysées à l'aide d'un microscope confocal (Olympus IX81). L'image intégrale de la coupe a été reconstituée en utilisant le logiciel XuvTools.

La tumorigenèse a été appréciée à l'aide de différentes analyses :
- Détermination de la localisation des foyers tumoraux par analyse histologique ;
- Mesure du volume tumoral à partir des images reconstruites en 3 dimensions ;
- Détermination de l'index prolifératif au sein des foyers tumoraux par détection du marqueur Ki67 ;
- Détermination de l'index de vascularisation des foyers tumoraux par détection de marqueurs d'angiogenèse ;
- Détermination de l'index de mort cellulaire au sein des foyers tumoraux par détection des événements de mort cellulaire (fragmentation de l'ADN, nécrose, relargage du cytochrome c, activation des protéases pro-apoptotiques) ;
- Analyse du transcriptome et du protéome des tumeurs extraites in *situ ;*
- Etude du comportement cellulaire après remise en culture.

Les embryons ont également été analysés à l'aide d'un ultramicroscope de marque LaVision Biotec. Les embryons ont été scannées et la tumeur restituée en 3D, permettant une analyse de volume et de localisation anatomique.

### RESULTATS

Selon le protocole expérimental décrit ci-dessus, des cellules cancéreuses humaines pulmonaires (lignée A549), de mélanome (lignée A375P), de glioblastome (lignée U251), de médulloblastome (lignée DEV) et de cancer de sein (lignée MDA MB 436), exprimant la GFP ou marquées par un colorant vital, ont été greffées dans un embryon de poulet au stade HH14. Des expériences complémentaires ont été réalisées à des stades plus précoces, HH10, HH11 et HH13.

### Exemple hors invention :

Les cellules de mélanomes ont été greffées au niveau du toit dorsal du tube neural, entre les somites 18 à 24.

48 heures après la greffe, les cellules de mélanomes forment des amas tumoraux au niveau sous-cutané ainsi que dans le mésenchyme bordant le tube neural. (Figure 4)

### Exemples selon l'invention :

Les cellules de glioblastome et de médulloblastome ont été greffées sur une zone allant de la crête neurale cervicale (en regard des somites 1 à 4), jusqu'aux tissus cérébraux bordant les ventricules cérébraux des différents territoires du cerveau.

48 heures après la greffe, les cellules de glioblastome et de médulloblastome forment des amas tumoraux dans le cerveau, ainsi que dans le tissu bordant les ventricules cérébraux. Ces tumeurs s'établissent dans le cerveau, de façon comparable aux tumeurs observées chez les patients. (Figure 5). Les cellules cancéreuses migrent dans le cerveau pour établir de nouveaux foyers.

Les cellules cancéreuses pulmonaires ont été greffées dans le cerveau, en différents endroits des territoires cérébraux. Ces cellules ont également été greffées dans le mésenchyme latéral en regard des crêtes neurales vagales et troncales (somites 4 à 24).

48 heures après la greffe, les cellules cancéreuses pulmonaires greffées au niveau des somites forment des amas tumoraux dans la corne ventrale du tube neural et son territoire latéral adjacent. (Figure 6 et figure 7A). Les cellules cancéreuses greffées dans les tissus cérébraux établissent des masses tumorales dans le cerveau, à partir desquelles se développent des métastases qui colonisent de nouvelles zones du cerveau ainsi que des territoires plus rostraux de l'embryon.

La figure 7B illustre la greffe de cellules tumorales pulmonaires dans des zones couvrant les sites présomptifs principaux de métastases des cancers pulmonaires humains :
- tissu périorbitaire,
- premier arc branchial,
- ébauche hépatique, et
- ébauche des membres - sclérotome/dermamyotome.

Les cellules ainsi greffées forment des amas tumoraux dans le cartilage et les os de la face (greffes périorbitaires et dans le premier arc branchial), dans le foie embryonnaire (greffe dans l'ébauche hépatique) et dans les tissus dérivant des somites tels que le tissu osseux (greffe dans le sclérotome/dermamyotome). Ces greffes sont dites 'hétérotopiques' puisque les tumeurs se forment dans des tissus différents de ceux dont elles sont originaires.

Les cellules cancéreuses de sein ont été greffées dans le cerveau, en différents endroits. 48h après la greffe, des amas tumoraux sont formés dans les tissus cérébraux. Un deuxième foyer de migration plus rostral que les sites de greffes est présent en superficie du cerveau, à proximité de la couche cutanée (Figure 8A).

Les cellules cancéreuses de sein ont été greffées dans le cerveau d'embryons à différents stades de développement : HH10, HH13 et HH14. Des foyers tumoraux ont été observés dans les tissus cérébraux sur chacun des embryons greffés, environ 48 heures après la greffe.

La figure 8B illustre la greffe de cellules tumorales mammaires dans des zones couvrant les sites présomptifs principaux de métastases des cancers mammaires humains :
- tissu périorbitaire,
- premier arc branchial,
- ébauche hépatique,
- ébauche des membres - sclérotome/dermamyotome.

Les cellules greffées forment des amas tumoraux dans le cartilage et les os de la face (greffes périorbitaires et dans le premier arc branchial), dans le foie embryonnaire (greffe dans l'ébauche hépatique) et dans les tissus dérivant des somites tels que le tissu osseux (greffe dans le sclérotome/dermamyotome). Ces greffes sont dites 'hétérotopiques' puisque les tumeurs se forment dans des tissus différents de ceux dont elles sont originaires.

Pour chaque type de cellules cancéreuses, plusieurs quantités de cellules cancéreuses greffées ont été testées, notamment des quantités de 1000 cellules, 3000 cellules et 10.000 cellules par greffon. Des formations de tumeurs ont été observées à chacune de ces concentrations, après 24 et 48h de développement de l'embryon greffé dans l'œuf après la greffe.

### REFERENCES BIBLIOGRAPHIQUES

US 6,228,345
WO 2015/074050
US 2013/0171680
Hamburger V., Hamilton H.L. A series of normal stages in the development of the chick embryo. J Morphol. 1951 Jan;88(1):49-92.
Hagedorn M, Javerzat S, Gilges D, Meyre A, de Lafarge B, Eichmann A, Bikfalvi A. Accessing key steps of human tumor progression in vivo by using an avian embryo model. Proc Natl Acad Sci U S A. 2005 Feb 1;102(5):1643-8.
Carter R, Mullassery D, See V, Theocharatos S, Pizer B, Losty PD, Jesudason E, Moss DJ. Exploitation of chick embryo environments to reprogram MYCN-amplified neuroblastoma cells to a benign phenotype, lacking detectable MYCN expression. Oncogenesis. 2012 Aug 27;1:e24.
Busch C, Krochmann J, Drews U. The chick embryo as an experimental system for melanoma cell invasion. PLoS One. 2013;8(1):e53970.
Cage TA, Louie JD, Liu SR, Alvarez-Buylla A, Gupta N, Hyer J. Distinct patterns of human medulloblastoma dissemination in the developing chick embryo nervous system. Clin Exp Metastasis. 2012 Apr;29(4):371-80.
Kulesa PM, Kasemeier-Kulesa JC, Teddy JM, Margaryan NV, Seftor EA, Seftor RE, Hendrix MJ. Reprogramming metastatic melanoma cells to assume a neural crest cell-like phenotype in an embryonic microenvironment. Proc Natl Acad Sci U S A. 2006 Mar 7;103(10):3752-7. Epub 2006 Feb 27.
Boulland JL, Halasi G, Kasumacic N, Glover JC. Xenotransplantation of human stem cells into the chicken embryo. J Vis Exp. 2010 Jul 11;(41).

## Revendications

1. Embryon de gallinacé dans lequel des cellules cancéreuses ont été greffées au sein des tissus de l'embryon, **caractérisé en ce que** :
- l'embryon est à un stade de développement compris entre le stade HH10 et le stade HH25 au moment de la greffe,
- lesdites cellules cancéreuses ne sont pas introduites dans la lumière du tube neural,
- lesdites cellules cancéreuses ne sont ni des cellules de neuroblastome, ni des cellules de mélanome,
- lesdites cellules se reproduisent et forment des tumeurs au sein de l'embryon, à un site d'implantation distinct du site de greffe, et
- ledit gallinacée est un poulet (*gallus gallus*) ou une caille (*Coturnix japonica*)*.*

2. Embryon de gallinacé selon la revendication 1, **caractérisé en ce que** l'embryon est à un stade de développement compris entre les stades HH10 et HH18 au moment de la greffe, ou entre les stades HH12 et HH16 au moment de la greffe, ou entre les stades HH13 et HH15 au moment de la greffe.

3. Embryon de gallinacé selon l'une des revendications 1 à 2, **caractérisé en ce que** l'embryon est incubé pendant au moins 24 heures après la greffe.

4. Embryon de gallinacé selon l'une des revendications 1 à 3, **caractérisé en ce que** les cellules cancéreuses sont greffées en une quantité d'au moins 1000 cellules par greffe.

5. Embryon de gallinacé selon l'une des revendications 1 à 4, **caractérisé en ce que** les cellules cancéreuses greffées sont des cellules humaines issues d'une tumeur de patient.

6. Embryon de gallinacé selon l'une des revendications 1 à 5, **caractérisé en ce que** les cellules cancéreuses greffées sont marquées par un colorant ou expriment une protéine marqueur.

7. Embryon de gallinacé selon l'une des revendications 1 à 6, **caractérisé en ce que** les cellules cancéreuses sont greffées dans un premier tissu distinct du tissu d'implantation où se forment les tumeurs, la greffe dans le premier tissu orientant les cellules cancéreuses greffées vers le tissu d'implantation où les tumeurs s'établissent.

8. Embryon de gallinacé selon l'une des revendications 1 à 7, **caractérisé en ce que** les cellules cancéreuses sont greffées dans le tube neural entre les somites 1 à 24 et/ou dans les tissus cérébraux.

9. Embryon de gallinacé selon l'une des revendications 1 à 8, **caractérisé en ce que** les cellules cancéreuses sont greffées dans les tissus cérébraux.

10. Embryon de gallinacé selon l'une des revendications 1 à 9, **caractérisé en ce que** les cellules cancéreuses greffées sont choisies parmi le groupe constitué des cellules issues de tumeurs cérébrales primaires ou secondaires, des cellules cancéreuses pulmonaires et des cellules de cancer du sein.

11. Embryon de gallinacé selon la revendication 8, **caractérisé en ce que** les cellules cancéreuses greffées sont issues de tumeurs cérébrales primaires ou secondaires, et sont greffées dans le tube neural entre les somites 1 à 4, et/ou dans les tissus cérébraux.

12. Procédé de préparation d'un embryon de gallinacé dans lequel des cellules cancéreuses ont été greffées puis ont formé des tumeurs au sein dudit embryon, comprenant les étapes suivantes :
• greffe de cellules cancéreuses au sein des tissus d'un embryon de gallinacé, lesdites cellules cancéreuses n'étant pas introduites dans la lumière du tube neural ; et
• incubation de l'embryon greffé pendant au moins 24 heures, lesdites cellules cancéreuses se reproduisant et formant des tumeurs au sein de l'embryon, dans un site d'implantation distinct du site de greffe ;
**caractérisé en ce que** :
ledit gallinacée est un poulet (*gallus gallus*) ou une caille (*Coturnix japonica*)*,*
l'embryon est à un stade de développement compris entre le stade HH10 et le stade HH25 au moment de la greffe, et
lesdites cellules cancéreuses n'étant ni des cellules de neuroblastome ni des cellules de mélanome.

13. Procédé pour le suivi d'un patient présentant une tumeur, comprenant :
a) la préparation d'un premier embryon greffé selon le procédé de la revendication 12, avec des cellules cancéreuses issues dudit patient à un instant T₁, et l'appréciation de la tumorigenèse des tumeurs se développant dans ce premier embryon,
b) la préparation d'un second embryon greffé selon le procédé de la revendication 12, avec des cellules cancéreuses issues dudit patient à un instant T₂, et l'appréciation de la tumorigenèse des tumeurs se développant dans ce second embryon,
c) la comparaison entre la tumorigenèse des tumeurs se développant dans le premier embryon greffé et dans le second embryon greffé.

14. Procédé pour le criblage de molécules thérapeutiques destinées au traitement d'un cancer, consistant en les étapes suivantes :
a) préparation d'un embryon greffé selon le procédé de la revendication 12 ;
b) administration à cet embryon greffé d'une molécule thérapeutique candidate;
c) appréciation de la malignité des cellules cancéreuses présentes dans cet embryon greffé après administration de ladite molécule candidate.

15. Procédé de préparation de tumeurs composées de cellules cancéreuses, comprenant les étapes suivantes :
• préparation d'un embryon greffé selon le procédé de la revendication 12 ;
• prélèvement desdites tumeurs formées au sein de l'embryon.

## Patentansprüche

1. Hühnervogelembryo, in dessen Gewebe Krebszellen transplantiert wurden, **dadurch gekennzeichnet, dass**:
- der Embryo zum Zeitpunkt der Transplantation in einem Entwicklungsstadium zwischen dem HH10-Stadium und dem HH25-Stadium ist,
- die Krebszellen nicht in das Lumen des Neuralrohrs eingeführt werden,
- die Krebszellen weder Neuroblastomzellen noch Melanomzellen sind,
- die Zellen sich vermehren und Tumore innerhalb des Embryos an einer von der Transplantationsstelle unterschiedlichen Implantationsstelle bilden, und
- der Hühnervogel ein Huhn *(gallus gallus)* oder eine Wachtel *(Coturnix japonica)* ist.

2. Hühnervogelembryo nach Anspruch 1, **dadurch gekennzeichnet, dass** der Embryo zum Zeitpunkt der Transplantation in einem Entwicklungsstadium zwischen den Stadien HH10 und HH18 oder zum Zeitpunkt der Transplantation zwischen den Stadien HH12 und HH16 oder zum Zeitpunkt der Transplantation zwischen den Stadien HH13 und HH15 ist.

3. Hühnervogelembryo nach einem der Ansprüche 1 bis 2, **dadurch gekennzeichnet, dass** der Embryo nach der Transplantation mindestens 24 Stunden lang inkubiert wird.

4. Hühnervogelembryo nach einem der Ansprüche 1 bis 3, **dadurch gekennzeichnet, dass** die Krebszellen in einer Menge von mindestens 1000 Zellen pro Transplantation transplantiert werden.

5. Hühnervogelembryo nach einem der Ansprüche 1 bis 4, **dadurch gekennzeichnet, dass** die transplantierten Krebszellen menschliche Zellen aus einem Tumor eines Patienten sind.

6. Hühnervogelembryo nach einem der Ansprüche 1 bis 5, **dadurch gekennzeichnet, dass** die transplantierten Krebszellen mit einem Farbstoff markiert sind oder ein Markerprotein exprimieren.

7. Hühnervogelembryo nach einem der Ansprüche 1 bis 6, **dadurch gekennzeichnet, dass** die Krebszellen in ein erstes Gewebe transplantiert werden, das sich von dem Implantationsgewebe unterscheidet, in dem sich die Tumore bilden, wobei die Transplantation in das erste Gewebe die transplantierten Krebszellen in Richtung des Implantationsgewebes ausrichtet, in dem sich die Tumore ansiedeln.

8. Hühnervogelembryo nach einem der Ansprüche 1 bis 7, **dadurch gekennzeichnet, dass** die Krebszellen in das Neuralrohr zwischen den Somiten 1 bis 24 und/oder in das Hirngewebe transplantiert werden.

9. Hühnervogelembryo nach einem der Ansprüche 1 bis 8, **dadurch gekennzeichnet, dass** die Krebszellen in das Hirngewebe transplantiert werden.

10. Hühnervogelembryo nach einem der Ansprüche 1 bis 9, **dadurch gekennzeichnet, dass** die transplantierten Krebszellen aus der Gruppe ausgewählt sind, die aus Zellen aus primären oder sekundären Hirntumoren, Lungenkrebszellen und Brustkrebszellen besteht.

11. Hühnervogelembryo nach Anspruch 8, **dadurch gekennzeichnet, dass** die transplantierten Krebszellen aus primären oder sekundären Hirntumoren stammen und in das Neuralrohr zwischen den Somiten 1 bis 4 und/oder in das Hirngewebe transplantiert werden.

12. Verfahren zur Herstellung eines Hühnervogelembryos, in den Krebszellen transplantiert wurden, die dann Tumore in diesem Embryo gebildet haben, umfassend die folgenden Schritte:
• Transplantation von Krebszellen in das Gewebe eines Hühnervogelembryos, wobei die Krebszellen nicht in das Lumen des Neuralrohrs eingeführt werden; und
• Inkubation des transplantierten Embryos für mindestens 24 Stunden, wobei sich die Krebszellen vermehren und Tumore innerhalb des Embryos innerhalb einer von der Transplantationsstelle unterschiedlichen Implantationsstelle bilden;
**dadurch gekennzeichnet, dass**:
der Hühnervogel ein Huhn (*gallus gallus*) oder eine Wachtel (*Coturnix japonica*) ist,
der Embryo zum Zeitpunkt der Transplantation in einem Entwicklungsstadium zwischen dem HH10-Stadium und dem HH25-Stadium ist, und
die Krebszellen weder Neuroblastomzellen noch Melanomzellen sind.

13. Verfahren zur Überwachung eines Patienten mit einem Tumor, umfassend:
a) die Herstellung eines ersten transplantierten Embryos nach dem Verfahren von Anspruch 12 mit Krebszellen des Patienten zu einem Zeitpunkt T₁ und die Beurteilung der Tumorentstehung der sich in diesem ersten Embryo entwickelnden Tumore,
b) die Herstellung eines zweiten transplantierten Embryos nach dem Verfahren von Anspruch 12 mit Krebszellen des Patienten zu einem Zeitpunkt T₂ und die Beurteilung der Tumorentstehung der sich in diesem zweiten Embryo entwickelnden Tumore,
c) den Vergleich der Tumorentstehung der sich im ersten transplantierten Embryo und im zweiten transplantierten Embryo entwickelnden Tumore.

14. Verfahren zum Screening von therapeutischen Molekülen zur Behandlung von Krebs, bestehend aus den folgenden Schritten:
a) Herstellung eines transplantierten Embryos nach dem Verfahren von Anspruch 12;
b) Verabreichung eines therapeutischen Kandidatenmoleküls an diesen transplantierten Embryo;
c) Beurteilung der Malignität der in diesem transplantierten Embryo nach Verabreichung des Kandidatenmoleküls vorhandenen Krebszellen.

15. Verfahren zur Herstellung von Tumoren aus Krebszellen, umfassend die folgenden Schritte:
• Herstellung eines transplantierten Embryos nach dem Verfahren von Anspruch 12;
• Entnahme der innerhalb des Embryos gebildeten Tumore.

## Claims

1. A gallinaceous bird embryo into which cancer cells have been grafted within the tissues of the embryo, **characterized in that**:
- the embryo is at a developmental stage between stage HH10 and stage HH25 at the time of the graft,
- said cancer cells are not introduced into the lumen of the neural tube,
- said cancer cells are not neuroblastoma cells not melanoma cells,
- said cancer cells reproduce and form tumours in an implantation site within the embryo that is distinct from the grafting site, and
- said gallinaceous bird is a chicken *(gallus gallus)* or a quail *(Coturnix japonica).*

2. The gallinaceous bird embryo of claim 1, **characterized in that** the embryo is at a developmental stage between stages HH10 and HH18 at the time of the graft, or between stages HH12 and HH16 at the time of the graft, or between stages HH13 and HH15 at the time of the graft.

3. The gallinaceous bird embryo of one of claims 1 to 2, **characterized in that** the embryo is incubated for at least 24 hours after the graft.

4. The gallinaceous bird embryo of one of claims 1 to 3, **characterized in that** the cancer cells are grafted in a quantity of at least 1,000 cells per graft.

5. The gallinaceous bird embryo of one of claims 1 to 4, **characterized in that** the grafted cancer cells are human cells derived from a tumour from a patient.

6. The gallinaceous bird embryo of one of claims 1 to 5, **characterized in that** the grafted cancer cells are labelled with a dye or express a marker protein.

7. The gallinaceous bird embryo of one of claims 1 to 6, **characterized in that** the cancer cells are grafted into a first tissue distinct from the implantation tissue where the tumours form, the graft into the first tissue directing the grafted cancer cells towards the implantation tissue where the tumours develop.

8. The gallinaceous bird embryo of one of claims 1 to 7, **characterized in that** the cancer cells are grafted into the neural tube between somites 1 and 24 and/or into the brain tissues.

9. The gallinaceous bird embryo of one of claims 1 to 8, **characterized in that** the cancer cells are grafted into the brain tissues.

10. The gallinaceous bird embryo of one of claims 1 to 9, **characterized in that** the grafted cancer cells are selected from the group consisting of: melanoma cells, cells derived from primary or secondary brain tumours, lung cancer cells and breast cancer cells.

11. The gallinaceous bird embryo of claim 8, **characterized in that** the grafted cancer cells are derived from primary or secondary brain tumours, and are grafted into the neural tube between somites 1 and 4, and/or into the brain tissues.

12. A process for preparing a gallinaceous bird embryo into which cancer cells have been grafted and then have formed tumours within said embryo, comprising the following steps:
• grafting of cancer cells within the tissues of a gallinaceous bird embryo, wherein said cancer cells are not introduced into the lumen of the neural tube, and
• incubation of the grafted embryo for at least 24 hours, wherein said cancer cells reproduce and form tumours in an implantation site within the embryo that is distinct from the grafting site;
**characterized in that**:
- said gallinaceous bird is a chicken (*gallus gallus*) or a quail (*Coturnix japonica*),
- said embryo is at a developmental stage between stage HH10 and stage HH25 at the time of the graft, and
- said cancer cells are not neuroblastoma cells nor melanoma cells.

13. A process for monitoring a patient with a tumour, comprising:
a) preparation of a first grafted embryo according to the process of claim 12, with cancer cells from said patient at a time T₁, and assessment of the tumorigenesis of the tumours developing in this first embryo,
b) preparation of a second grafted embryo according to the process of claim 12, with cancer cells from said patient at a time T₂, and assessment of the tumorigenesis of the tumours developing in this second embryo,
c) comparison between the tumorigenesis of the tumours developing in the first grafted embryo and in the second grafted embryo.

14. A process for screening therapeutic molecules intended for the treatment of cancer, consisting of the following steps:
a) preparation of a grafted embryo according to the process of claim 12;
b) administration of a candidate therapeutic molecule to this grafted embryo;
c) assessment of the malignancy of the cancer cells present in this grafted embryo after administration of said candidate molecule.

15. A process for preparing tumours composed of cancer cells, comprising the following steps:
• preparation of a grafted embryo according to the process of claim 12,
• sampling of said tumours formed within the embryo.
